# EUROPEAN PATENT APPLICATION

(11) **EP 3 264 084 A1**
(43) Date of publication of application: **03.01.2018**
(21) Application number: 16755661.2
(22) Date of filing: 25.02.2016
(51) Int. Cl.: G01N 33/531, G01N 33/53, G01N 33/543

(54) **IMMUNOASSAY METHOD AND ASSAY REAGENT USED IN SAID METHOD**

(30) Priority: 25.02.2015 JP 2015035903
(71) Applicant: Sekisui Medical Co., Ltd., Tokyo 103-0027 (JP)
(72) Inventor: KOBAYASHI, Koji, Tokyo 103-0027 (JP); MATSUMOTO, Takuji, Tokyo 103-0027 (JP); YAMAMOTO, Mitsuaki, Tokyo 103-0027 (JP)
(74) Representative: Hoffmann Eitle
(86) International application number: PCT/JP2016/055728
(87) International publication number: WO 2016/136917

(57) **Abstract**

An object of the present invention is to provide a more accurate and sensitive measurement method lowering the possibility of occurrence of nonspecific reaction in immunological measurement of L-FABP. More specifically, an object is to provide a measurement method that is accurate and sensitive even when the concentration of an analyte is low (e.g., an L-FABP concentration is around the normal value). In an immunological measurement method of L-FABP using an anti-L-FABP antibody, nonspecific reaction can easily be suppressed by a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide even in highly sensitive measurement.

## Description

### TECHNICAL FIELD

The present invention relates to an immunological measurement method for L-FABP using an anti-L-FABP antibody and a measurement reagent used in the method.

### BACKGROUND ART

Fatty acid binding proteins (FABPs) are a group of proteins present in the cytosol and having a molecular weight of about 14 kilodalton and an ability to bind to a fatty acid and includes at least seven known molecular species such as a liver type (L-FABP), an intestinal type (I-FABP), a heart type (H-FABP), a brain type (B-FABP), a skin type (C-FABP/E-FABP), an adipocyte type (aP2), and a peripheral nerve cell type (myelin P2), and these are thought to be a family evolved from a common ancestral gene. While each type of FABP shows a specific tissue distribution, the name indicates the tissue in which the type was first found, and does not necessarily means that the type exists only in that tissue. For example, at least two types of FABP, the liver type (L-FABP) and the heart type (H-FABP) are expressed in the human kidney tissue and, among them, L-FABP is distributed in the proximal tubule, while H-FABP is mainly distributed in the distal tubule (Maatman et al., Biochemical Journal, vol.288, pp.285-290, 1992; Maatman et al., Biochemical Journal, vol.273, pp.759-766, 1991).

L-FABP is excreted into urine due to ischemia (blood flow failure) of the renal tubule and oxidative stress to the renal tubule before progression of tissue damage. Urinary L-FABP correlates with the degree of interstitial tubular disorder in a human renal biopsy tissue and is a marker reflecting tubular disorder (Kamijo et al., Am J Pathol, vol.165, No.4, pp.1243-1255, 2004). It has long been known that the progression and prognosis of kidney disease correlates with the degree of interstitial tubule damage rather than the degree of glomerular lesion (Risdon et al., Lancet, vol.2, pp.363-366, 1968), and urinary L-FABP is a marker of tubular damage and is useful for predicting the prognosis of kidney disease.

Patent Document 1 discloses a kidney disease examination method characterized by focusing on a relationship between the expression of L-FABP in the kidney tissue and the prognosis of kidney disease and detecting the fatty acid binding protein derived from the kidney tissue present in a test sample. More specifically, in a described example, urine collected from a renal disease patient was used as a sample to measure an amount of L-FABP leaked into the urine with sandwich ELISA, which is an immunological measurement method, by using an anti-mouse L-FABP polyclonal antibody. For an immunological measurement method, important issues include minimizing the amount of reaction system, reducing measurement time, improving measurement sensitivity, etc. and it is desirable to improve measurement sensitivity also in the measurement of L-FABP. However, detailed measurement conditions (conditions for sample processing and antigen-antibody reaction), detection sensitivity, measurement values, etc. are not described in Patent Document 1.

Methods of improving measurement sensitivity of immunological measurement method include a method of increasing the detection upper limit and a method of lowering the detection lower limit of the measurement range. While immunological measurement methods are characterized by high specificity and good sensitivity, it is also known that various kinds of interference disturb the detection of specific reaction between an antigen and an antibody. Nonspecific reaction due to interference impairs accuracy of measurement values and causes a problem that measurement values different from true values are obtained. To lower the detection lower limit so as to increase the sensitivity (to accurately perform measurement even in a low concentration range), nonspecific reaction as mentioned above must be suppressed.

Patent Document 2 discloses an immunological measurement method of performing an antigen-antibody reaction and/or a measurement in the presence of a polycarboxylic-acid-type surfactant for the purpose of suppressing nonspecific reaction and improving correlation with the measurement result of a conventional reagent particularly in a low concentration range.

Patent Document 3 discloses an immunological measurement method of allowing polyoxyethylene alkyl ether sulfate and polyoxyethylene sorbitan fatty acid ester to coexist in a reaction system for the purpose of suppressing variations in blank measurement values and lowering the blank measurement values so as to improve measurement accuracy in a low concentration range of a test substance.

### CITATION LIST

### PATENT LITERATURE

Patent Document 1: Japanese Laid-Open Patent Publication No. H11-242026
Patent Document 2: Japanese Laid-Open Patent Publication No. 2013-205408
Patent Document 3: WO 2010/113943

### SUMMARY OF INVENTION

### TECHNICAL PROBLEM

An object of the present invention is to provide a more accurate and sensitive measurement method lowering the possibility of occurrence of nonspecific reaction in immunological measurement of L-FABP. More specifically, an object is to provide a measurement method that is accurate and sensitive even when the concentration of an analyte is low (e.g., an L-FABP concentration is around the normal value).

### SOLUTION TO PROBLEM

As a result of intensive studies, the present inventors have found that nonspecific reaction can easily be suppressed even in highly sensitive measurement by allowing a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide to be present, in an immunological measurement method of L-FABP using an anti-L-FABP antibody.

In particular, the present invention has the following configuration.
[1] A method of detecting with an anti-L-FABP antibody L-FABP (liver-type fatty acid binding protein) in a sample, or a method of stabilizing a measurement value in a method of detecting with an anti-L-FABP antibody L-FABP (liver-type fatty acid binding protein) in a sample, comprising the step of: bringing
   a sample suspected of containing L-FABP,
   particles having an immobilized anti-L-FABP antibody,
   a compound having a partial structure of NH₂-C=N- in a molecule, and
   a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide, into contact with each other.
[2] The method according to item [1] above, wherein the compound having a partial structure of NH₂-C=N- in a molecule is one or two or more selected from a compound represented by Formula (1) or a salt or ester thereof and a compound represented by Formula (2) or a salt thereof:
   the compound of Formula (1) 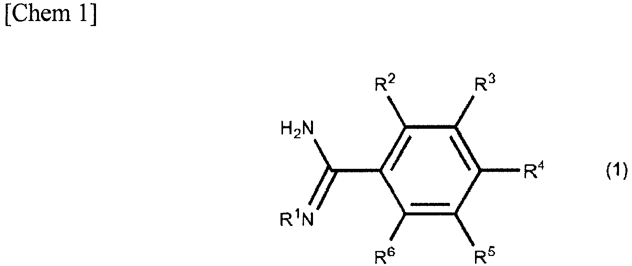 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or a salt or ester thereof; and
   the compound of Formula (2) 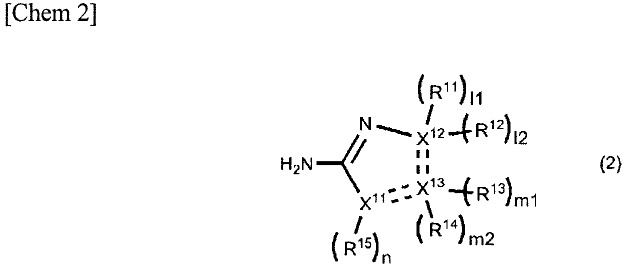
      [in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
      R¹⁵ is a hydrogen atom, a halogen atom, or an alkyl group having the carbon number of 1, 2, or 3 that may be branched,
      X¹¹ is a nitrogen atom or a sulfur atom,
      X¹² and X¹³ are each independently a carbon atom or a nitrogen atom, and
      l1, l2, m1, m2, and n are each independently 0 or 1,
      the double broken line between X¹¹ and X¹³ and the double broken line between X¹² and X¹³ are each independently a single bond or a double bond, wherein
      the values of l1, l2, m1, m2, and n as well as the bonds of the double broken line between X¹¹ and X¹³ and the double broken line between X¹² and X¹³ indicate values and bonds determined in conformity with the valences of X¹¹ to X¹³], or a salt thereof.
[3] The method according to item [2] above, wherein the compound represented by Formula (2) or a salt thereof is one or two or more selected from the following compound or a salt thereof:
   the compound of Formula (2) 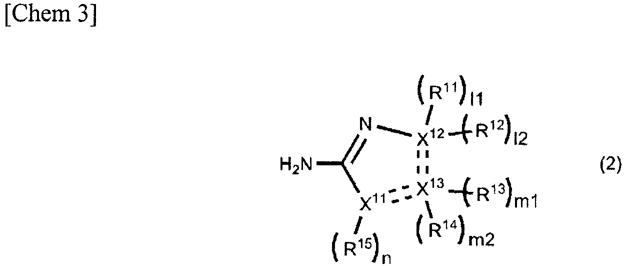
      [In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
      R¹⁵ is a hydrogen atom, a halogen atom, or an alkyl group having the carbon number of 1, 2, or 3 that may be branched], wherein
      in a combination of X¹¹ to X¹³, l1+l2, m1+m2, n (l1,l2, m1, m2, and n each independently represent 0 or 1), and double broken lines,
         (a) X¹¹ is a sulfur atom, X¹² and X¹³ are carbon atoms, 11+12 is 2, ml+m2 is 2, n is 0, and the double broken lines between X¹¹ and X¹³ and between X¹² and X¹³ are single bonds,
         (b) X¹¹ is a sulfur atom, X¹² and X¹³ are carbon atoms, 11+12 is 1, ml+m2 is 1, n is 0, the double broken line between X¹¹ and X¹³ is a single bond, and the double broken line between X¹² and X¹³ is a double bond,
         (c) X¹¹ is a nitrogen atom, X¹² and X¹³ are carbon atoms, l1+l2 is 2, m1+m2 is 2, n is 1, and the double broken lines between X¹¹ and X¹³ and between X¹² and X¹³ are single bonds,
         (d) X¹¹ is a nitrogen atom, X¹² and X¹³ are carbon atoms, 11+12 is 1, m1+m2 is 1, n is 1, the double broken line between X¹¹ and X¹³ is a single bond, and the double broken line between X¹² and X¹³ is a double bond,
         (e) X¹¹ and X¹² are nitrogen atoms, X¹³ is a carbon atom, l1+l2 is 1, m1+m2 is 1, n is 0, the double broken line between X¹¹ and X¹³ is a double bond, and the double broken line between X¹² and X¹³ is a single bond, or
         (f) X¹¹, X¹², and X¹³ are nitrogen atoms, l1+l2 is 0, m1+m2 is 0, n is 1, the double broken line between X11 and X13 is a single bond, and the double broken line between X¹² and X¹³ is a double bond, or a salt thereof.
[4] The method according to item [1] above, wherein the compound having a partial structure of NH₂-C=N- in a molecule is either benzamidine or 2-amino-2-thiazoline.
[5] The method according to any one of items [1] to [4] above, wherein
   at the step of bringing a sample suspected of containing L-FABP, an anti-L-FABP antibody, a compound having a partial structure of NH₂-C=N- in a molecule, and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide into contact with each other,
   the step of bringing the sample suspected of containing L-FABP, the compound, and the polypeptide into contact with each other is followed by the step of bringing the sample into contact with the anti-L-FABP antibody.
[6] The method according to item [5] above, wherein the concentration of the polypeptide at the step of bringing the sample suspected of containing L-FABP, the compound, and the polypeptide into contact with each other is 0.04 mmol/L to 1.36 mmol/L.
[7] The method according to any one of items [1] to [6] above, wherein the anti-L-FABP antibody is two or more monoclonal antibodies having recognition sites different from each other.
[8] The method according to any one of items [1] to [7] above, wherein the particles are latex particles.
[9] The method according to item [7] or [8] above, wherein the two or more anti-L-FABP monoclonal antibodies having recognition sites different from each other are respectively immobilized on the latex particles, and wherein L-FABP is detected by a latex turbidimetric immunoassay.
[10] The method according to item [7] above, wherein one monoclonal antibody of the two or more monoclonal antibodies having recognition sites different from each other is labeled with a labeling substance while the other monoclonal antibody or antibodies are immobilized on a solid phase or solid phases, and wherein L-FABP is detected by immunochromatography.
[11] The method according to any one of items [1] to [10] above, wherein the sample is urine, whole blood, serum, or plasma.
[12] A reagent for a particle immunoassay for detecting with an anti-L-FABP antibody L-FABP in a sample, comprising: an anti-L-FABP antibody; a compound having a partial structure of NH₂-C=N- in a molecule; and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.
[13] A method of avoiding nonspecific reaction in a method of detecting L-FABP around the normal value in a sample with a particle immunoassay, wherein a mixed liquid of a sample suspected of containing L-FABP and a measurement reagent contains a compound having a partial structure of NH₂-C=N- in a molecule and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.
[14] A method of stabilizing a measurement value in a method of detecting L-FABP around the normal value in a sample with a particle immunoassay, wherein a mixed liquid of a sample suspected of containing L-FABP and a measurement reagent contains a compound having a partial structure of NH₂-C=N- in a molecule and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.

### ADVANTAGEOUS EFFECTS OF INVENTION

According to the present invention, since nonspecific reaction derived from a reaction system can be suppressed even in a highly sensitive immunological measurement method, a more accurate and sensitive immunological measurement method can be provided. For example, a specimen can accurately be measured in urinary L-FABP measurement around the normal value (e.g., 2.2±2.3 µg/gCR. according to the package insert of Renapro (registered trademark) L-FABP test TMB).

### DESCRIPTION OF EMBODIMENTS

The present invention will now be described in detail.

### (Sample)

Examples of the sample used in the present invention include urine, blood (whole blood, plasma, or serum), kidney tissue, extract from kidney tissue, etc. Among them, urine is a particularly preferred sample. Any samples are usable as long as the samples are suspected of containing L-FABP, including a sample derived from a healthy subject, a sample derived from a patient, a sample derived from a person suspected of having a disease, etc.

### (Anti-L-FABP Antibody)

For the anti-L-FABP antibody used in the present invention, a native L-FABP purified from organs, cells, body fluid, etc. can be prepared as an immunogen (antigen). L-FABP is mainly distributed in the liver or the kidney and therefore can be purified and isolated from these organs etc. Additionally, it is known that L-FABP is highly homologous among humans, mice, pigs, cows, and rats and has a homology of 90% or more at the amino acid level and, therefore, for example, mouse L-FABP can be used as an antigen for acquiring an antibody binding to human L-FABP.

Purification of native L-FABP can be performed in accordance with a method described in Kelvin et al. (J. Biol. Chem., vol.263, pp.15762-15768, 1988) etc. In particular, after homogenizing an excised organ, a cytoplasmic fraction acquired by ultracentrifugation is fractionated by gel filtration, anion exchange chromatography, etc., and the fraction containing L-FABP is selected by using a molecular weight or fatty acid binding activity as an index, isolated, and purified. The selected fraction is subjected to SDS-polyacrylamide electrophoresis to confirm that the purified protein forms a single band and is further purified if needed. For the purified protein, the amino-acid composition and the N-terminal amino-acid sequence are determined and compared with the reported composition and sequence so as to confirm that the protein is the intended molecular species.

L-FABP used as an antigen may be a recombinant protein produced by a genetic engineering technique. Since the amino acid sequence and the gene sequence of L-FABP are already reported (Veerkamp and Maatman, Prog. Lipid Res., vol.34, pp.17-52, 1995), for example, a primer can be designed based on these sequences for cloning of cDNA from an appropriate cDNA library etc. by a PCR (polymerase chain reaction) method. This cDNA can be used for performing gene recombination so as to prepare recombinant L-FABP. Additionally, a fragment of L-FABP or a synthetic peptide etc. having a partial sequence thereof can be bound to a carrier macromolecular substance (BSA, hemocyanin, etc.) as needed and used as the antigen.

An antibody specifically binding to L-FABP may be any of antisera, polyclonal antibodies, monoclonal antibodies, etc.

The antibody preferably has a high specificity and, for example, in the case of an anti-L-FABP antibody, desirably, the antibody is substantially not cross-reactive with H-FABP. To acquire an antibody with a higher specificity, a highly purified and highly pure antigen is desirably used. When an antibody is prepared, a warm-blooded animal other than human is immunized by inoculating the purified antigen prepared as described above. Examples of the warm-blooded animal to be immunized other than human include mammals (rabbits, sheep, rats, mice, guinea pigs, horses, pigs, etc.) and birds (chickens, ducks, geese etc.). In the case of rabbits, for example, about 100 µg to 1 mg of the antigen emulsified in about 1 mL of saline and Freund's complete adjuvant is inoculated subcutaneously in the dorsum or the palm of a hind foot and, from the second time, the adjuvant is replaced with Freund's incomplete adjuvant, and the antigen is inoculated three to eight times at intervals of two to four weeks for immunization, so as to use the antibody produced about 7 to 12 days after the final inoculation. In the case of mice, 10 to 30 µg/animal of the antigen is usually inoculated subcutaneously, intraperitoneally, or intravenously three to eight times at intervals of about two weeks for immunization, so as to use the antibody produced about two to four days after the final inoculation.

Polyclonal antibodies can be prepared by collecting blood from the animal immunized as described above, separating serum (antiserum), and recovering an Ig fraction from the acquired antiserum. For example, polyclonal IgG can be acquired by recovering an IgG fraction from the antiserum by affinity chromatography using a Protein G column etc.

Monoclonal antibody is produced by a hybridoma acquired by fusing antibody-producing cells collected from an immunized animal with immortalized cells. Mice and rats are preferably used as immunized animals for the monoclonal antibodies. The hybridoma can be produced in accordance with the method of Kohler and Milstein (Kohler and Milstein, Nature, vol.256, pp.495-897, 1975) as follows. Antibody-producing cells (such as splenocytes or lymph node cells) from an animal immunized as described above are collected and fused with appropriate immortalized cells. For example, cell lines of myeloma cells (NSI-Ag 4/1, Sp2/O-Agl4, etc.) are preferably used as immortalized cells. The myeloma cells are preferably nonsecretory cells not producing antibodies or immunoglobulin H or L chains by themselves. The myeloma cells preferably have a selection marker so that unfused myeloma cells and fused hybridomas may be screened in a selection medium. For example, for the selection marker, cell lines having 8-azaguanine resistance (hypoxanthine-guanine-phosphoribosyltransferase deficiency), thymidine kinase deficiency, etc. are often used. The cell fusion is performed by adding an appropriate fusion promoter such as polyethylene glycol. The cell fusion is preferably performed at a ratio of about 10 antibody-producing cells per immortalized cell, and can preferably be performed at a cell density of about 10⁶ cells/mL of the antibody-producing cells.

The cells subjected to the fusion treatment are appropriately diluted and then cultured in a selection medium for one to two weeks. For example, when myeloma cells resistant to 8-azaguanine are used and cultured in the HAT (hypoxanthine, aminopterin, thymidine) medium, the unfused myeloma cells die, and the unfused antibody-producing cells also die because of limited division cycle; however, only the fused cells can continue to undergo division and survive in the selection medium. After culturing in the selection medium, the supernatant is subjected to, for example, ELISA with an antigen immobilized on a solid phase, so as to detect the presence/absence of the intended antibody, and cloning can be performed by a limiting dilution method to select a hybridoma producing a monoclonal antibody recognizing the intended antigen. At the time of selection, the hybridoma is selected that produces the monoclonal antibody having desired properties such as antibody titer, antibody class, subclass, affinity for antigen, specificity, epitope, etc. IgG is generally preferable as the class of monoclonal antibody.

The monoclonal-antibody-producing hybridoma is intraperitoneally implanted in an animal of the same species as the animal used for immunization and, after elapse of a certain period, the ascites can be collected from the animal to isolate the intended monoclonal antibody. Alternatively, the hybridoma can be cultured in an appropriate animal cell culture medium, and the monoclonal antibody can be isolated from the culture solution. Once the intended hybridoma is acquired, the gene encoding the monoclonal antibody can be acquired from the hybridoma to express and produce the intended monoclonal antibody in an appropriate host (e.g., silkworm, etc.) by a common gene recombination technique. Separation and purification of the antibody can be performed in accordance with, for example, a usual purification method achieved by combining ammonium sulfate precipitation, gel chromatography, ion exchange chromatography, affinity chromatography, etc. as needed.

The anti-L-FABP antibody used in the present invention may be a known antibody or an antibody to be developed in the future. Although not particularly limited, usable commercially available anti-L-FABP antibodies include C-4 (catalog No. sc-374537), F-9 (catalog No. sc-271591) of Santa Cruz Biotechnology, 328607 (catalog No. MAB2964) of R&D systems, L2B10 (Catalog No. HA2049-IA) of Hycult biotech, 2G4 (Catalog No. LS-B3001) of Lifespan Biosciences, etc. These antibodies bind to polypeptides of the internal region, the N-terminal region, etc. of the human-derived L-FABP protein and, even when the antibodies bind to the internal region of the L-FABP molecule, L-FABP can be detected with higher sensitively and higher specificity by using a compound having a partial structure of NH₂-C=N- and a cyclic structure in a molecule of the present invention.

"Antibody" in the present invention includes not only intact immunoglobulin molecules but also antibody fragments or antibody derivatives having an antigen binding abilities known in the art, such as Fab, Fab'₂, CDR, a humanized antibody, a multifunctional antibody, and a single chain antibody (ScFv).

### (Detection)

The method of the present invention for detecting L-FABP using an anti-L-FABP antibody is an immunological measurement method. More specifically, examples thereof include, but not limited to, a particle immunoagglutination measurement method such as a latex turbidimetric immunoassay (LTIA), a chemiluminescence detection method, and immunochromatography (lateral-flow type, flow-through type). Among them, an immunological measurement method not including a step for B/F separation (homogeneous immunoassay method) is more preferable. It is noted that when LTIA is described as a measurement method in this description, the detection method thereof may be achieved by using any of known detection methods such as measurement of change in transmitted light (absorbance), measurement of change in scattered light, and measurement of change in particle diameter. Furthermore, the term "detection" or "measurement" must be construed in the broadest sense including the proof of the presence and/or the quantification of L-FABP, and must not be construed in a limited manner.

### (Insoluble Carrier)

An insoluble carrier used in the present invention can be an insoluble carrier made of a polymeric base material such as polystyrene resin, an inorganic base material such as glass, a polysaccharide base material such as cellulose and agarose, etc. and is not particularly limited in terms of the shape thereof, and any shapes can be selected in accordance with the measurement method to be adopted, including a bead or particle shape (e.g., latex particles, metal colloid particles), a plate or sheet shape (e.g., a porous membrane), etc.

Examples of the particles include latex particles mainly composed of polystyrene generally used in the particle immunoagglutination measurement method as well as particles containing a styrene-butadiene copolymer, a (meth)acrylic acid ester polymer, etc. as a base material. Particles made of metal colloid, gelatin, liposome, microcapsule, silica, alumina, carbon black, metallic compound, metal, ceramics, or magnetic material are also usable. For the carrier particles used in the present invention, one and the same kind of material or two or more kinds of materials can be used.

The particle diameter of the carrier particles is preferably 0.15 to 0.45 µm, more preferably 0.2 to 0.4 µm. Two or more kinds of the carrier particles different in average particle diameter can be used in combination.

The porous membrane can be a known membrane and can be made of any material. Examples of the material of the porous membrane include, but not limited to, polyethylene, polyethylene terephthalate, nylons, glass, polysaccharides such as cellulose and cellulose derivatives, ceramics, etc. Specifically, the examples include glass fiber filter paper, cellulose filter paper, etc. sold by Millipore, Toyo Roshi, Whatman, etc.

### (Immobilization of Antibody to Insoluble Carrier)

A method for immobilizing an anti-L-FABP antibody on an insoluble carrier is not particularly limited, and any known method can be used. If an anti-L-FABP antibody is immobilized on particles, this is achieved by using, for example, a physical adsorption method using physical adsorption caused by mixing particles and the antibody, or a chemical binding method using a coupling agent such as carbodiimide to chemically bind a carboxy or an amino group on the particle surface to an antibody molecule. The antibody molecules may be immobilized on the particles via spacer molecules. Furthermore, after binding the antibody to another protein such as albumin by using the chemical binding method, the protein may physically or chemically be immobilized on the particles.

If an anti-L-FABP antibody is immobilized on a porous membrane, the antibody can be immobilized, for example, by applying a certain amount of a solution containing the antibody into a shape of a line, a dot, a specific symbol such as + to the porous membrane.

In this description, the "insoluble carrier" is referred to as a "solid phase", and allowing, or a state of allowing, the insoluble carrier to physically or chemically support an antigen or an antibody is referred to as "immobilization", "immobilized", "solid-phased", "sensitization", or "adsorption" in some cases.

### (Labeled Antibody)

Examples of a labeling substance for labeling the antibody include, for example, an enzyme, a fluorescent substance, a chemiluminescent substance, biotin, avidin, a radioactive isotope, gold colloid particles, or colored latex particles. A method of binding the labeling substance and the antibody can be methods such as a glutaraldehyde method, a maleimide method, a pyridyl disulfide method, or a periodic acid method available to those skilled in the art. Both the labeling substance and the binding method are not limited to those described above and any known methods can be used. With regard to the detection of the label, for example, when an enzyme such as peroxidase or alkaline phosphatase is used as the labeling substance, the enzymatic activity can be measured by using a specific substrate of the enzyme (e.g., 1,2-phenylenediamine or 3,3',5,5'-tetramethylbenzidine when the enzyme is horseradish peroxidase, or p-nitrophenyl phosphate in the case of alkaline phosphatase) and, when biotin is used as the labeling substance, avidin labeled at least with a labeling substance other than biotin is typically reacted therewith.

### (Compound Having Partial Structure of NH₂-C=N- and Cyclic Structure in Molecule)

A compound having a partial structure of NH₂-C=N- in a molecule of the present invention can also have a cyclic structure and is represented by Formula (1) and Formula (2) (sometimes referred to as a "compound of Formula (1)" and a "compound of Formula (2)" in the description).

The compound of Formula (1) is a compound of 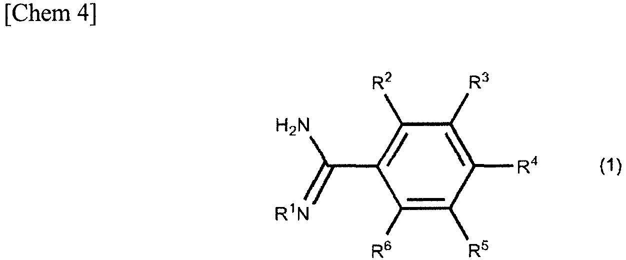 [in Formula (1), R¹ is a hydrogen atom, a hydroxyl group, or an alkyl group having the carbon number of 1 to 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or a salt or ester thereof.

The compound of Formula (2) is a compound of 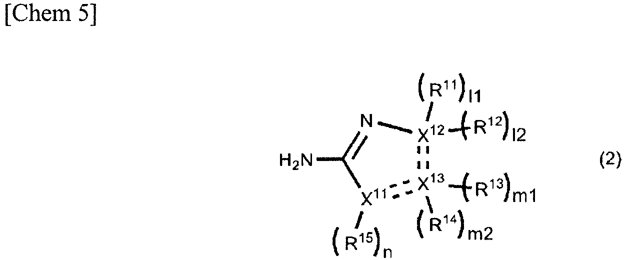
[in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
R¹⁵ is a hydrogen atom, a halogen atom, or an alkyl group having the carbon number of 1, 2, or 3 that may be branched,
X¹¹ is a nitrogen atom or a sulfur atom,
X¹² and X¹³ are each independently a carbon atom or a nitrogen atom, and
11,12, m1, m2, and n are each independently 0 or 1,
the double broken line between X¹¹ and X¹³ and the double broken line between X¹² and X¹³ are each independently a single bond or a double bond, wherein
the values of 11,12, m1, m2, and n as well as the bonds of the double broken line between X¹¹ and X¹³ and the double broken line between X¹² and X¹³ indicate values and bonds determined in conformity with the valences of X¹¹ to X¹³], or a salt thereof.

Additionally, the compound of Formula (2) is a compound of 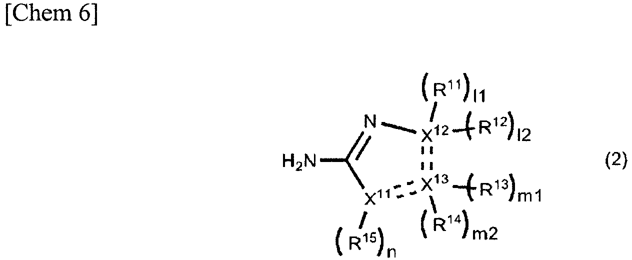
[In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
R¹⁵ is a hydrogen atom, a halogen atom, or an alkyl group having the carbon number of 1, 2, or 3 that may be branched], wherein
in a combination of X¹¹ to X¹³, l1+l2, m1+m2, n (11,12, m1, m2, and n each independently represent 0 or 1), and double broken lines,
   (a) X¹¹ is a sulfur atom, X¹² and X¹³ are carbon atoms, l1+l2 is 2, m1+m2 is 2, n is 0, and the double broken lines between X¹¹ and X¹³ and between X¹² and X¹³ are single bonds,
   (b) X¹¹ is a sulfur atom, X¹² and X¹³ are carbon atoms, 11+12 is 1, m1+m2 is 1, n is 0, the double broken line between X¹¹ and X¹³ is a single bond, and the double broken line between X¹² and X¹³ is a double bond,
   (c) X¹¹ is a nitrogen atom, X¹² and X¹³ are carbon atoms, 11+12 is 2, m1+m2 is 2, n is 1, and the double broken lines between X¹¹ and X¹³ and between X¹² and X¹³ are single bonds,
   (d) X¹¹ is a nitrogen atom, X¹² and X¹³ are carbon atoms, l1+l2 is 1, m1+m2 is 1, n is 1, the double broken line between X¹¹ and X¹³ is a single bond, and the double broken line between X¹² and X¹³ is a double bond,
   (e) X¹¹ and X¹² are nitrogen atoms, X¹³ is a carbon atom, l1+l2 is 1, m1+m2 is 1, n is 0, the double broken line between X¹¹ and X¹³ is a double bond, and the double broken line between X¹² and X¹³ is a single bond, or
   (f) X¹¹, X¹², and X¹³ are nitrogen atoms, l1+12 is 0, m1+m2 is 0, n is 1, the double broken line between X¹¹ and X¹³ is a single bond, and the double broken line between X¹² and X¹³ is a double bond, or a salt thereof.

Examples of the compound represented by Formula (1) include a benzamidine derivative and examples of the compound represented by Formula (2) include an aminothiazole derivative, an aminotriazole derivative, an aminotetrazole derivative, and an aminoimidazole derivative. The salt of each compound having a partial structure of NH₂-C=N- in a molecule can be selected as needed from hydrochloride, sulfate, nitrate, hydrobromate, hydrofluoride, borofluoride, oxalate, lactate, adipate, tartrate, hydroiodide, toluenesulfonate, malonate, bicarbonate, etc. without particular limitation in consideration of the effect of the present invention as well as easiness of handling, availability, etc.

More specific examples of the compound of Formula (1) include benzamidine hydrochloride (CAS No. 1670-14-0), benzamidine hydrochloride hydrate (CAS No. 206752-36-5), 4-fluorobenzamidoxime (CAS No. 69113-32-2), and 4-chlorobenzamidine hydrochloride (CAS No. 14401-51-5). More specific examples of the compound of Formula (2) include aminothiazoline (CAS No. 1779-81-3), 2-amino-2-thiazoline hydrochloride (CAS No. 3882-98-2), pseudothiohydantoin (CAS No. 556-90-1), 2-amino-5-bromothiazole hydrobromide (CAS No. 61296-22-8), 2-amino-4,5-dimethylthiazole hydrobromide (CAS No. 7170-76-5), and 2,4-diamino-5-phenylthiazole monohydrobromide (CAS No.:6020-54-8). Among them, benzamidine hydrochloride and 2-amino-2-thiazoline hydrochloride are particularly preferable.

Examples of the compound having a partial structure of NH₂-C=N- in a molecule of the present invention include a compound having a guanidino group (H₂N-(C=NH)-NH-R). R in the compound or a salt thereof is a hydrogen atom, an amino group, a phenyl group, an alkyl group, a carboxyalkyl group, a substituted or unsubstituted aminoalkyl group, a substituted or unsubstituted aminocarboxyalkyl group, a substituted or unsubstituted aminocarbonylalkyl group, a sulfonyl group, or the like. Examples of the alkyl group can include an alkyl group having the carbon number of 1, 2, 3, 4, 5, or 6 on a straight chain or a branched chain, and the alkyl group having the carbon number of 1, 2, 3, 4, 5, or 6 on a straight chain or a branched chain is specifically exemplified by a methyl group, an ethyl group, a propyl group, a butyl group, an isobutyl group, a sec-butyl group, a tert-butyl group, a pentyl group, a hexyl group, etc.

Examples of the compound having the guanidino group include a guanidine salt or a derivative thereof. More specific examples include guanidine (CAS No. 113-00-8), guanidine sulfamate (CAS No. 50979-18-5), aminoguanidine (CAS No. 79-17-4).

The compounds having a partial structure of NH₂-C=N- in a molecule of the present invention may be used solely or may be used as a combination of two or more compounds.

### (BPF)

A polypeptide consisting of amino acids No. 419 to No. 607 of the amino acid sequence of DnaK, a heat shock protein (HSP), derived from E. coli (also referred to as Blocking Peptide Fragment; hereinafter sometimes also referred to as "BPF") is a peptide having a molecular weight of about 22000 disclosed as a novel substance for blocking in an immunological measurement method in WO 2005/003155 and Polymer Preprints, Japan Vol. 55, No. 2, 5211-5212 (2006), and is also commercially available.

The BPF used for a method of the present invention etc. can be prepared in accordance with the description of WO 2005/003155. Alternatively, a commercially available product (manufactured by TOYOBO CO., LTD., Catalog No. BPF-301) may be used.

### (Method of Bringing Sample Suspected of Containing L-FABP, Anti-L-FABP Antibody, Compound Having Partial Structure of NH₂-C=N- in Molecule, and BPF into Contact with Each Other)

The step of bringing a sample suspected of containing L-FABP, an anti-L-FABP antibody, a compound having a partial structure of NH₂-C=N- in a molecule, and BPF into contact with each other may be achieved by using any method as long as a step of bringing the sample suspected of containing L-FABP, the compound, and BPF into contact with each other is followed by the step of bringing the sample into contact with the anti-L-FABP antibody.

For example, the method of bringing the sample suspected of containing L-FABP into contact with the compound and/or BPF can be, for example, a method of mixing a liquid reagent containing the compound and/or BPF with the sample. Another method can be a method of supplying the sample to an insoluble carrier such as a porous membrane infiltrated with the compound and/or BPF so that the contact occurs. It is noted that the compound and BPF may not necessarily simultaneously be brought into contact with the sample suspected of containing L-FABP. Those skilled in the art can appropriately achieve the setting in consideration of the configuration of the measurement reagent etc.

Furthermore, L-FABP in the sample is by a known appropriate method brought into contact with an anti-L-FABP antibody immobilized on an insoluble carrier, after, or at the same time as, the contact with the compound and/or BPF.

Examples of the method of bringing the compound having a partial structure of NH₂-C=N- in a molecule of the present invention into contact with a sample include a method in which the compound is brought into contact as a diluent of a sample, an extraction solution of a sample, or a preservation solution, a development solution, etc. of a sample.

A preferable range of the concentration of the compound having a partial structure of NH₂-C=N- in a molecule of the present invention may be 50 mmol/L to 1000 mmol/L, 50 mmol/L to 500 mmol/L, 50 mmol/L to 600 mmol/L, 100 mmol/L to 900 mmol/L, 200 mmol/L to 800 mmol/L, 300 mmol/L to 600 mmol/L, 300 mmol/L to 550 mmol/L, 300 mmol/L to 500 mmol/L, and 350 mmol/L to 450 mmol/L, and is preferably 50 mmol/L to 500 mmol/L. The optimum concentration of each of the compounds having a partial structure of NH₂-C=N- in a molecule to be used can experimentally be obtained as described in this description.

A preferable range of the concentration of BPF may be 0.01 to 5 %, 0.02 to 5 %, 0.03 to 5 %, 0.04 to 5 %, 0.05 to 5 %, 0.1 to 5 %, 0.2 to 5 %, 0.3 to 5 %, 0.4 to 5 %, 0.75 to 5 %, 1 to 5 %, 1 to 4 %, and 1 to 3 %, and can be exemplified by preferably 0.05 to 5 %, more preferably 0.075 to 5 %, further preferably 0.1 to 3 % (all w/v%). Those skilled in the art can experimentally determine the optimum concentration of BPF.

For example, when BPF-301 having a molecular weight of about 22000 is used, the concentration may be 0.0045 mmol/L to 2.27 mmol/L, 0.0090 mmol/L to 2.27 mmol/L, 0.013 mmol/L to 2.27 mmol/L, 0.018 mmol/L to 2.27 mmol/L, 0.022 mmol/L to 2.27 mmol/L, 0.045 mmol/L to 2.27 mmol/L, 0.090 mmol/L to 2.27 mmol/L, 0.13 mmol/L to 2.27 mmol/L, 0.18 mmol/L to 2.27 mmol/L, 0.22 mmol/L to 2.27 mmol/L, 0.45 mmol/L to 2.27 mmol/L, 0.45 mmol/L to 1.81 mmol/L, and 0.45 mmol/L to 1.36 mmol/L, and is preferably 0.022 mmol/L to 2.27 mmol/L, more preferably 0.034 mmol/L to 2.27 mmol/L, and further preferably 0.045 mmol/L to 1.36 mmol/L.

### (Measurement Kit)

Constituents of a measurement kit provided according to the present invention other than the compound having a partial structure of NH₂-C=N- in a molecule of the present invention are not particularly limited as long as L-FABP can immunologically be measured. The measurement kit will hereinafter be described by taking immunochromatography and LTIA as an example.

### (Immunochromatography)

Typical immunochromatography is configured by using a test strip equipped with "1. a sample-supply portion", "2. a portion of retaining a labeled antibody (a labeled antibody-retaining portion)", and "3. a portion of immobilizing an antibody for capturing a complex formed by the labeled antibody and the L-FABP antibody (a capture-antibody portion)" in order in a direction of development of a solution containing a sample on a sheet-like insoluble carrier such as a porous membrane, such that the sample solution continuously moves due to capillarity. In the case of immunochromatography, the measurement kit at least includes the test strip as described above.

Specifically, when a predetermined amount of a sample containing L-FABP is added to the sample-supply portion, the sample enters the labeled- retaining portion due to capillarity, and L-FABP and the labeled antibody bind together to form a complex. When the complex developed through the membrane enters the capture-antibody portion, the complex is captured by the antibody (capture antibody) immobilized on the membrane to form a ternary complex of [the capture antibody]-[L-FABP]-[the labeled antibody]. The label can be detected by an arbitrary method (e.g., an agglutination image in the case of a label that can be made visible such as gold colloid particles, or a coloring reaction due to addition of a substrate in the case of an enzyme), so as to detect the presence of L-FABP.

For example, the compound having a partial structure of NH₂-C=N- in a molecule and/or BPF of the present invention can preliminarily be added to a sample diluent etc. or can preliminarily be contained in the sample-supply portion or the labeled-retaining portion and thereby can be brought into contact with L-FABP in the sample.

### (Latex Turbidimetric Immunoassay)

In the case of latex turbidimetric immunoassay, the measurement kit includes at least latex particles having an antibody immobilized thereon. For the antibody used in latex turbidimetric immunoassay, any combination of "two monoclonal antibodies having different recognition sites for antigens", "polyclonal antibodies", or "monoclonal antibody and polyclonal antibody" can be used. In this case, the latex particles are the insoluble carrier having an antibody immobilized thereon and the labeling substance at the same time.

The latex particles used for these measurement reagents can appropriately be selected in terms of particle diameter and material so as to acquire desired performance such as improved sensitivity. The latex particles may be any particles suitable for supporting the antibody. For example, the particles may contain polystyrene, a styrene-sulfonic acid (salt) copolymer, a styrene-methacrylic acid copolymer, an acrylonitrile-butadiene-styrene copolymer, a vinyl chloride- acrylic acid ester copolymer, a vinyl acetate-acrylic acid ester copolymer, etc., as a base material. Although the shape of the latex particles is not particularly limited, preferably, the average particle diameter is sufficiently large so that aggregates generated as a result of agglutination reaction between the antibody on the latex particle surfaces and L-FABP can be detected with the naked eye or optically. Particles made of material such as metal colloid, gelatin, liposome, microcapsule, silica, alumina, carbon black, metal compound, metal, ceramics, or magnetic material can be used instead of the latex particles.

A typical measurement kit for LTIA used in clinical examination is usually provided in a form of a first reagent and a second reagent. Both or one of two kinds of the latex particles having the immobilized antibody described above can be contained in the first reagent or the second reagent. Although it is generally preferable that both of the latex particles having the immobilized antibody be contained in the second reagent, one and the other of the particles can be contained in the first reagent and the second reagent, respectively.

The compound having a partial structure of NH₂-C=N- in a molecule of the present invention and/or BPF are preferably contained in the first reagent. By mixing with the sample the first reagent containing the compound having a partial structure of NH₂-C=N- in a molecule of the present invention and/or BPF, L-FABP in the sample contacts with the compound having a partial structure of NH₂-C=N- in a molecule of the present invention and/or BPF.

In addition to those described above, the kit of the present invention appropriately includes a buffer component (buffer solution). The buffer solution usable in the present invention may be any commonly used buffer solutions including tris-hydrochloric acid, boric acid, phosphoric acid, acetic acid, citric acid, succinic acid, phthalic acid, glutaric acid, maleic acid, glycine, and salts thereof, and Good's buffers such as MES, Bis-Tris, ADA, PIPES, ACES, MOPSO, BES, MOPS, TES, and HEPES, for example.

The kit of the present invention also includes saccharides, proteins, etc. as needed for the purpose of improving measurement sensitivity and suppressing nonspecific reaction. Examples thereof include components promoting antigen-antibody reactions (polymeric compounds such as polyethylene glycol, polyvinyl pyrrolidone, phospholipid polymers, etc.), proteins and peptides (albumin, casein, etc.), amino acids, sugars (sucrose, cyclodextrin, etc.), and preservatives (sodium azide, ProClin 300, etc.).

Although native L-FABP derived from tissues such as liver and kidney can be used as a standard substance (L-FABP standard substance) in sample measurement, the standard substance may be a recombinant protein produced by a genetic engineering technique. Since the amino acid sequence and the gene sequence of L-FABP have already been reported (Veerkamp and Maatman, Prog. Lipid Res., vol.34, pap.17-52, 1995), for example, a primer can be designed based on these sequences for cloning of cDNA from an appropriate cDNA library etc. by a PCR (polymerase chain reaction) method. This cDNA can be used for preparing recombinant L-FABP by gene recombination techniques. For the standard substance, it is more preferable to use the recombinant protein with stable structure.

### EXAMPLES

Examples of the present invention will hereinafter be described to more specifically describe the present invention; however, the present invention is not limited thereto and can variously be applied without departing from the technical idea of the present invention.

### (Anti-L-FABP Antibody-Immobilized Latex Particle Suspension)

### (1) Preparation of Clone L Antibody-Immobilized Latex Particle Suspension

To 13 mL of a 20 mmol/L Tris buffer solution (pH 8.5) containing 0.64 mg/mL of anti-L-FABP antibody Clone L (manufactured by CMIC HOLDINGS Co., Ltd.), 13 mL of a 1 % latex particle (manufactured by SEKISUI CHEMICAL CO., LTD.) suspension having an average particle diameter of 0.212 µm was added and stirred at 4 °C for two hours. This was followed by addition of 13 mL of a 20 mmol/L Tris buffer solution (pH 8.5) containing 0.5 % BSA and stirring at 4 °C for one hour. Subsequently, dialysis with a 5 mmol/L MOPS buffer solution (pH 7.0) was performed to acquire a Clone L antibody-immobilized latex particle suspension.

### (2) Preparation of Clone 1 Antibody-Immobilized Latex Particle Suspension

To 8 mL of a 5 mmol/L Tris buffer solution (pH 7.5) containing 0.64 mg/mL of anti-L-FABP antibody Clone 1 (manufactured by CMIC HOLDINGS Co., Ltd.), 8 mL of a 1 % latex particle (manufactured by SEKISUI CHEMICAL CO., LTD.) suspension having an average particle diameter of 0.315 µm was added and stirred at 4 °C for two hours. This was followed by addition of 8 mL of a 5 mmol/L Tris buffer solution (pH 7.5) containing 0.5 % BSA and stirring at 4 °C for one hour. Subsequently, dialysis with a 5 mmol/L MOPS buffer solution (pH 7.0) was performed to acquire a Clone 1 antibody-immobilized latex particle suspension.

### (L-FABP Standard Substance)

The L-FABP standard substance was acquired by gene recombination as described in Patent Document 1.

### (L-FABP Reference Measurement Method: Reference Method)

An ELISA-based in vitro diagnostic product (Renapro (registered trademark) L-FABP test TMB) was used for a reference method.

### (First Control Reagent: Also Serving as Standard Substance Diluent)

300 mmol/L Bis-Tris buffer solution (pH 6.9)
100 mmol/L NaCl
0.47 to 0.57 % Lipidure-BL403SE
(Second Reagent)
5 mmol/L MOPS buffer solution (pH 7.0)
3.75 Abs/mL Clone L antibody-immobilized latex particle suspension^{(*)}
3.75 Abs/mL Clone 1 antibody-immobilized latex particle suspension^{(*)}
(*) Abs denotes the absorbance at 280 nm.

### (Standard Solution)

The L-FABP standard substance was adjusted to a desired concentration by using the standard substance diluent and was used as a standard solution.

### (Frozen-Thawed Urine)

Partial urine (n=32; the measurement value in the reference method is 0.3 to 4.6 ng/mL) frozen and stored at -30 °C after collection was thawed only once and used for measurement.

### (Measurement Conditions of LTIA)

(1) Analyzing device: Hitachi 7170 Automatic Analyzer (manufactured by Hitachi High-Technologies Corporation)
(2) Sample amount and reagent amounts: 3 µL of sample, 150 µL of the first reagent, 50 µL of the second reagent
(3) Reaction time (reaction temperature): 5 minutes (37 °C) for the first reagent, 5 minutes (37 °C) for the second reagent
(4) Photometric point and photometric object: absorbance changes between immediately after addition of the second reagent and 5 minutes after the addition

### [Example 1] Confirmation of Nonspecific Reaction-Suppression Effect by BPF on Specimen Having L-FABP Concentration around Normal Value, Part 1

A nonspecific reaction-suppression effect in an L-FABP concentration range around the normal value was compared when no protein (Control Example 1), BSA (Comparative Example 1), or 0.1% BPF (Example 1) was added to the first reagent.

### 1. Operation

### (1) Example 1

The frozen-thawed urine was employed as a sample and L-FABP in the sample was measured by using the first control reagent containing 390 mmol/L of the compound of Formula (1) (benzamidine hydrochloride) and 0.1 % BPF, and the second reagent.

### (2) Control Example 1

The frozen-thawed urine was employed as a sample and L-FABP in the sample was measured by using the first control reagent containing 390 mmol/L of the compound of Formula (1) (benzamidine hydrochloride), and the second reagent.

### (3) Comparative Example 1

The frozen-thawed urine was employed as a sample and L-FABP in the sample was measured by using the first control reagent containing 390 mmol/L of the compound of Formula (1) (benzamidine hydrochloride) and 0.1% BSA, and the second reagent.

### 2. Results

The net absorbance of the samples measured in the respective tests of Example 1, Control Example A, and Comparative Example 1 was converted into L-FABP concentration by using a calibration curve created by using the standard solution as samples. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied at L-FABP concentrations around the normal value by the least-squares method and shown in Table 1.

**[Table 1]**

| | Added protein | R² | Regression equation |
|---|---|---|---|
| Example 1 | 0.1% B P F | 0.891 | y=1. 512x-1. 232 |
| Control Example 1 | None | 0. 024 | y=0.500x+5.193 |
| Comp.Ex.1 | 0.1% B S A | 0.007 | y=0. 437x+9. 774 |

| | | | |
|---|---|---|---|
| Comp.Ex.1: Comparative Example 1 | | | |

R² between the measurement value and the reference measurement value in Control Example 1 was as low as 0.024 and thus it was confirmed that the condition of Control Example 1 is not applicable to the measurement using urine, a specimen of clinical examination, as a sample. R² between the measurement value and the reference measurement value in Comparative Example 1 was 0.007, which was worse than that of Control Example in which no protein was added. On the other hand, R² between the measurement value and the reference measurement value in Example 1 was 0.891 and the correlation was favorable. From the above, it was confirmed that in the measurement of L-FABP using the latex turbidimetric immunoassay, the specimen having an L-FABP concentration around the normal value can accurately be measured by adding BPF.

### [Examples 2 to 5] Confirmation of Nonspecific Reaction-Suppression Effect on Specimen Having L-FABP Concentration around Normal Value by BPF, Part 2

The nonspecific reaction-suppression effect in an L-FABP concentration range around the normal value was compared when 390 mmol/L of the compound of formula (1) (benzamidine hydrochloride) and 0.01 to 1 % BPF (Examples 2 to 5) were added to the first reagent.

### 1. Operation

For a specimen, partial urine (n=10; the measurement value in the reference method is 0.6 to 4.9 ng/mL) frozen and stored at -30 °C after collection was thawed only once and used for measurement. To the first reagent, BPF was added in the amount shown in Table 2. Except these points, Example 2 was performed in the same manner as Example 1.

### 2. Results

The net absorbance of the samples measured in the tests of Examples 2 to 5 was converted into L-FABP concentration by using a calibration curve created by using the standard solution as samples. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied at L-FABP concentrations around the normal value by the least-squares method and shown in Table 2.

**[Table 2]**

| | BPF(%) | R² |
|---|---|---|
| Ex.2 | 0.01 | 0.924 |
| Ex.3 | 0.05 | 0.896 |
| Ex.4 | 0.5 | 0.926 |
| Ex.5 | 1 | 0.939 |

| | | |
|---|---|---|
| Ex.: Example | | |

R² between the measurement value and the reference measurement value in Examples 2 to 5 were 0.896 to 0.939 and the correlation was favorable.

### [Examples 6 to 9] Confirmation of Nonspecific Reaction-Suppression Effect on Specimen Having L-FABP Concentration around Normal Value by BPF, Part 3

The nonspecific reaction-suppression effect in an L-FABP concentration range around the normal value was compared when 390 mmol/L of the compound of formula (2) (2-amino-2-thiazoline hydrochloride) and 0.01 to 1 % BPF (Examples 6 to 9) were added to the first reagent.

### 1. Operation

For a specimen, partial urine (n=10; the measurement value in the reference method is 0.6 to 4.9 ng/mL) frozen and stored at -30 °C after collection was thawed only once and used for measurement. To the first reagent, the compound of the formula (2) (2-amino-2-thiazoline hydrochloride) and BPF in the amount shown in Table 3 were added. Except these points, Example 3 was performed in the same manner as Example 1.

### 2. Results

The net absorbance of the samples measured in the tests of Examples 6 to 9 was converted into L-FABP concentration by using a calibration curve created by using the standard solution as samples. By plotting the L-FABP concentrations of the samples obtained by using the reference method (reference measurement values) on the x-axis and the L-FABP concentrations obtained from the respective tests (test measurement values) on the y-axis, the correlation of the test measurement values with the reference measurement values was studied at L-FABP concentrations around the normal value by the least-squares method and shown in Table 3.

**[Table 3]**

| | BPF(%) | R² |
|---|---|---|
| Ex.6 | 0.01 | 0.665 |
| Ex.7 | 0.05 | 0.606 |
| Ex.8 | 0.5 | 0.579 |
| Ex.9 | 1 | 0.673 |

| | | |
|---|---|---|
| Ex.: Example | | |

R² between the measurement value and the reference measurement value in Examples 6 to 9 was 0.579 to 0.673. The correlation was favorable as compared to no addition of BPF (Control Example 1) or addition of 0.1 % BSA (Comparative Example 1).

### INDUSTRIAL APPLICABILITY

According to the present invention, nonspecific reaction derived from the reaction system can be suppressed even in a highly sensitive immunological measurement method and, therefore, a more accurate and sensitive immunological measurement method can be provided. For example, a specimen can accurately be measured around the normal value in urinary L-FABP measurement (e.g., 2.2±2.3 µg/gCR. from the package insert of Renapro (registered trademark) L-FABP test TMB).

## Claims

1. A method of detecting with an anti-L-FABP antibody L-FABP (liver-type fatty acid binding protein) in a sample, comprising the step of: bringing
a sample suspected of containing L-FABP,
particles having an immobilized anti-L-FABP antibody,
a compound having a partial structure of NH₂-C=N- in a molecule, and
a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide, into contact with each other.

2. The method according to claim 1, wherein the compound having a partial structure of NH₂-C=N- in a molecule is one or two or more selected from a compound represented by Formula (1) or a salt or ester thereof and a compound represented by Formula (2) or a salt thereof:
the compound of Formula (1) 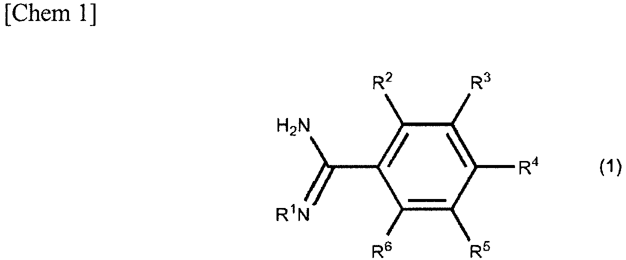 [in Formula (1), R¹ is a hydrogen atom, a hydroxyl group, or an alkyl group having the carbon number of 1, 2, or 3 that may be branched, and R² to R⁶ each independently represent a hydrogen atom, a halogen atom, an alkyl group having the carbon number of 1, 2, or 3 that may be branched, a hydroxyl group, a carboxy group, an amino group, or -SR⁷ (R⁷ represents a hydrogen atom, a hydroxyl group, or an alkyl group having the carbon number of 1, 2, or 3 that may be branched and, when a plurality of R⁷s are present, R⁷s may be the same groups as or different groups from each other)] or a salt or ester thereof; and
the compound of Formula (2) 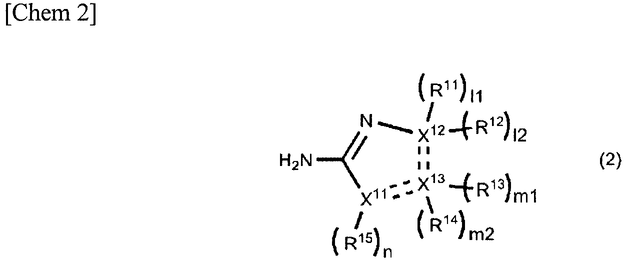
[in Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
R¹⁵ is a hydrogen atom, a halogen atom, or an alkyl group having the carbon number of 1, 2, or 3 that may be branched,
X¹¹ is a nitrogen atom or a sulfur atom,
X¹² and X¹³ are each independently a carbon atom or a nitrogen atom, and
11,12, m1, m2, and n are each independently 0 or 1,
the double broken line between X¹¹ and X¹³ and the double broken line between X¹² and X¹³ are each independently a single bond or a double bond, wherein
the values of l1,l2, m1, m2, and n as well as the bonds of the double broken line between X¹¹ and X¹³ and the double broken line between X¹² and X¹³ indicate values and bonds determined in conformity with the valences of X¹¹ to X¹³], or a salt thereof.

3. The method according to claim 2, wherein the compound represented by Formula (2) or a salt thereof is one or two or more selected from the following compound or a salt thereof:
the compound of Formula (2) 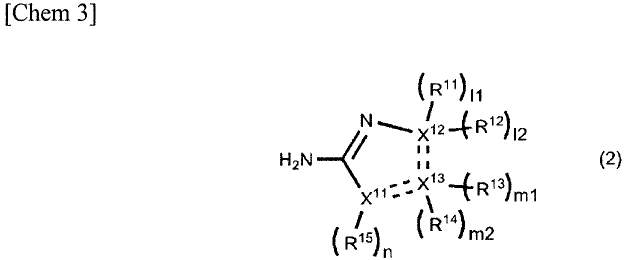
[In Formula (2), R¹¹ to R¹⁴ each independently represent a hydrogen atom, a halogen atom, an alkyl group having the carbon number of 1, 2, or 3 that may be branched, an amino group, a phenyl group that may be substituted with a halogen atom, or -SR¹⁶ (R¹⁶ represents a hydrogen atom, a hydroxyl group, or an alkyl group having the carbon number of 1, 2, or 3 that may be branched and, when a plurality of R¹⁶s are present, R¹⁶s may be the same groups as or different groups from each other), wherein R¹¹ and R¹² present in the same molecule may form a carbonyl group together and R¹³ and R¹⁴ present in the same molecule may form a carbonyl group together,
R¹⁵ is a hydrogen atom, a halogen atom, or an alkyl group having the carbon number of 1, 2, or 3 that may be branched], wherein
in a combination of X¹¹ to X¹³, l1+l2, m1+m2, n (11, 12, m1, m2, and n each independently represent 0 or 1), and double broken lines,
(a) X¹¹ is a sulfur atom, X¹² and X¹³ are carbon atoms, 11+12 is 2, m1+m2 is 2, n is 0, and the double broken lines between X¹¹ and X¹³ and between X¹² and X¹³ are single bonds,
(b) X¹¹ is a sulfur atom, X¹² and X¹³ are carbon atoms, 11+12 is 1, ml+m2 is 1, n is 0, the double broken line between X¹¹ and X¹³ is a single bond, and the double broken line between X¹² and X¹³ is a double bond,
(c) X¹¹ is a nitrogen atom, X¹² and X¹³ are carbon atoms, 11+12 is 2, ml+m2 is 2, n is 1, and the double broken lines between X¹¹ and X¹³ and between X¹² and X¹³ are single bonds,
(d) X¹¹ is a nitrogen atom, X¹² and X¹³ are carbon atoms, l1+l2 is 1, m1+m2 is 1, n is 1, the double broken line between X¹¹ and X¹³ is a single bond, and the double broken line between X¹² and X¹³ is a double bond,
(e) X¹¹ and X¹² are nitrogen atoms, X¹³ is a carbon atom, 11+12 is 1, ml+m2 is 1, n is 0, the double broken line between X¹¹ and X¹³ is a double bond, and the double broken line between X¹² and X¹³ is a single bond, or
(f) X¹¹, X¹², and X¹³ are nitrogen atoms, l1+l2 is 0, m1+m2 is 0, n is 1, the double broken line between X¹¹ and X¹³ is a single bond, and the double broken line between X¹² and X¹³ is a double bond, or a salt thereof.

4. The method according to claim 1, wherein the compound having a partial structure of NH₂-C=N- in a molecule is either benzamidine or 2-amino-2-thiazoline.

5. The method according to any of claims 1 to 4, wherein
at the step of bringing a sample suspected of containing L-FABP, an anti-L-FABP antibody, a compound having a partial structure of NH₂-C=N- in a molecule, and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide into contact with each other,
the step of bringing the sample suspected of containing L-FABP, the compound, and the polypeptide into contact with each other is followed by the step of bringing the sample into contact with the anti-L-FABP antibody.

6. The method according to claim 5, wherein the concentration of the polypeptide at the step of bringing the sample suspected of containing L-FABP, the compound, and the polypeptide into contact with each other is 0.04 mmol/L to 1.36 mmol/L.

7. The method according to any of claims 1 to 6, wherein the anti-L-FABP antibody is two or more monoclonal antibodies having recognition sites different from each other.

8. The method according to claims 1 to 7, wherein the particles are latex particles.

9. The method according to claim 7 or 8, wherein the two or more anti-L-FABP monoclonal antibodies having recognition sites different from each other are respectively immobilized on the latex particles, and wherein L-FABP is detected by a latex turbidimetric immunoassay.

10. The method according to claim 7, wherein one monoclonal antibody of the two or more monoclonal antibodies having recognition sites different from each other is labeled with a labeling substance while the other monoclonal antibody or antibodies are immobilized on a solid phase or solid phases, and wherein L-FABP is detected by immunochromatography.

11. The method according to any of claims 1 to 10, wherein the sample is urine, whole blood, serum, or plasma.

12. A reagent for a particle immunoassay for detecting with an anti-L-FABP antibody L-FABP in a sample, comprising: an anti-L-FABP antibody; a compound having a partial structure of NH₂-C=N- in a molecule; and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.

13. A method of avoiding nonspecific reaction in a method of detecting L-FABP around the normal value in a sample with a particle immunoassay, wherein a mixed liquid of a sample suspected of containing L-FABP and a measurement reagent contains a compound having a partial structure of NH₂-C=N- in a molecule and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.

14. A method of stabilizing a measurement value in a method of detecting L-FABP around the normal value in a sample with a particle immunoassay, wherein a mixed liquid of a sample suspected of containing L-FABP and a measurement reagent contains a compound having a partial structure of NH₂-C=N- in a molecule and a polypeptide consisting of amino acids No. 419 to No. 607 of the amino acid sequence of DnaK, a heat shock protein (HSP), derived from E. coli as set forth in SEQ ID NO: 1 or a polypeptide having at least 90 % sequence identity with the polypeptide.


SEQUENCE LISTING

<110>  SEKISUI MEDICAL CO., LTD.

<120>  Immunoassay and reagent therefor

<130>  15P00236

<150>  JP2015-035903
<151>  2015-02-25

<160>  1

<170>  PatentIn version 3.5

<210>  1
<211>  638
<212>  PRT
<213>  Escherichia coli

<220>
<221>  DnaK
<222>  (1)..(638)

<220>
<221>  BPF
<222>  (419)..(607)

<400>  1

Met Gly Lys Ile Ile Gly Ile Asp Leu Gly Thr Thr Asn Ser Cys Val
1               5                   10                  15

Ala Ile Met Asp Gly Thr Thr Pro Arg Val Leu Glu Asn Ala Glu Gly
            20                  25                  30

Asp Arg Thr Thr Pro Ser Ile Ile Ala Tyr Thr Gln Asp Gly Glu Thr
        35                  40                  45

Leu Val Gly Gln Pro Ala Lys Arg Gln Ala Val Thr Asn Pro Gln Asn
    50                  55                  60

Thr Leu Phe Ala Ile Lys Arg Leu Ile Gly Arg Arg Phe Gln Asp Glu
65                  70                  75                  80

Glu Val Gln Arg Asp Val Ser Ile Met Pro Phe Lys Ile Ile Ala Ala
                85                  90                  95

Asp Asn Gly Asp Ala Trp Val Glu Val Lys Gly Gln Lys Met Ala Pro
            100                 105                 110

Pro Gln Ile Ser Ala Glu Val Leu Lys Lys Met Lys Lys Thr Ala Glu
        115                 120                 125

Asp Tyr Leu Gly Glu Pro Val Thr Glu Ala Val Ile Thr Val Pro Ala
    130                 135                 140

Tyr Phe Asn Asp Ala Gln Arg Gln Ala Thr Lys Asp Ala Gly Arg Ile
145                 150                 155                 160

Ala Gly Leu Glu Val Lys Arg Ile Ile Asn Glu Pro Thr Ala Ala Ala
                165                 170                 175

Leu Ala Tyr Gly Leu Asp Lys Gly Thr Gly Asn Arg Thr Ile Ala Val
            180                 185                 190

Tyr Asp Leu Gly Gly Gly Thr Phe Asp Ile Ser Ile Ile Glu Ile Asp
        195                 200                 205

Glu Val Asp Gly Glu Lys Thr Phe Glu Val Leu Ala Thr Asn Gly Asp
    210                 215                 220

Thr His Leu Gly Gly Glu Asp Phe Asp Ser Arg Leu Ile Asn Tyr Leu
225                 230                 235                 240

Val Glu Glu Phe Lys Lys Asp Gln Gly Ile Asp Leu Arg Asn Asp Pro
                245                 250                 255

Leu Ala Met Gln Arg Leu Lys Glu Ala Ala Glu Lys Ala Lys Ile Glu
            260                 265                 270

Leu Ser Ser Ala Gln Gln Thr Asp Val Asn Leu Pro Tyr Ile Thr Ala
        275                 280                 285

Asp Ala Thr Gly Pro Lys His Met Asn Ile Lys Val Thr Arg Ala Lys
    290                 295                 300

Leu Glu Ser Leu Val Glu Asp Leu Val Asn Arg Ser Ile Glu Pro Leu
305                 310                 315                 320

Lys Val Ala Leu Gln Asp Ala Gly Leu Ser Val Ser Asp Ile Asp Asp
                325                 330                 335

Val Ile Leu Val Gly Gly Gln Thr Arg Met Pro Met Val Gln Lys Lys
            340                 345                 350

Val Ala Glu Phe Phe Gly Lys Glu Pro Arg Lys Asp Val Asn Pro Asp
        355                 360                 365

Glu Ala Val Ala Ile Gly Ala Ala Val Gln Gly Gly Val Leu Thr Gly
    370                 375                 380

Asp Val Lys Asp Val Leu Leu Leu Asp Val Thr Pro Leu Ser Leu Gly
385                 390                 395                 400

Ile Glu Thr Met Gly Gly Val Met Thr Thr Leu Ile Ala Lys Asn Thr
                405                 410                 415

Thr Ile Pro Thr Lys His Ser Gln Val Phe Ser Thr Ala Glu Asp Asn
            420                 425                 430

Gln Ser Ala Val Thr Ile His Val Leu Gln Gly Glu Arg Lys Arg Ala
        435                 440                 445

Ala Asp Asn Lys Ser Leu Gly Gln Phe Asn Leu Asp Gly Ile Asn Pro
    450                 455                 460

Ala Pro Arg Gly Met Pro Gln Ile Glu Val Thr Phe Asp Ile Asp Ala
465                 470                 475                 480

Asp Gly Ile Leu His Val Ser Ala Lys Asp Lys Asn Ser Gly Lys Glu
                485                 490                 495

Gln Lys Ile Thr Ile Lys Ala Ser Ser Gly Leu Asn Glu Asp Glu Ile
            500                 505                 510

Gln Lys Met Val Arg Asp Ala Glu Ala Asn Ala Glu Ala Asp Arg Lys
        515                 520                 525

Phe Glu Glu Leu Val Gln Thr Arg Asn Gln Gly Asp His Leu Leu His
    530                 535                 540

Ser Thr Arg Lys Gln Val Glu Glu Ala Gly Asp Lys Leu Pro Ala Asp
545                 550                 555                 560

Asp Lys Thr Ala Ile Glu Ser Ala Leu Thr Ala Leu Glu Thr Ala Leu
                565                 570                 575

Lys Gly Glu Asp Lys Ala Ala Ile Glu Ala Lys Met Gln Glu Leu Ala
            580                 585                 590

Gln Val Ser Gln Lys Leu Met Glu Ile Ala Gln Gln Gln His Ala Gln
        595                 600                 605

Gln Gln Thr Ala Gly Ala Asp Ala Ser Ala Asn Asn Ala Lys Asp Asp
    610                 615                 620

Asp Val Val Asp Ala Glu Phe Glu Glu Val Lys Asp Lys Lys
625                 630                 635